# EUROPÄISCHE PATENTSCHRIFT

(11) **EP 1 890 731 B1**
(45) Veröffentlichungstag und Bekanntmachung des Hinweises auf die Patenterteilung: **09.02.2011**
(21) Anmeldenummer: 06723684.4
(22) Anmeldetag: 24.03.2006
(51) Int. Cl.: A61L 2/232, A61L 2/16, B05D 3/14, A61L 101/20

(54) **VERFAHREN ZUR HERSTELLUNG EINER ANTIMIKROBIELL WIRKENDEN BESCHICHTUNG AUF EINER TECHNISCHEN OBERFLÄCHE**
METHOD FOR PRODUCING AN ANTIMICROBIAL COATING ON A TECHNICAL SURFACE
PROCEDE DE FABRICATION D'UN REVETEMENT A EFFET ANTIMICROBIEN SUR UNE SURFACE TECHNIQUE

(30) Priorität: 13.06.2005 DE 102005027347
(43) Veröffentlichungstag der Anmeldung: 27.02.2008
(73) Patentinhaber: Fraunhofer-Gesellschaft zur Förderung der angewandten Forschung e.V., 80686 München (DE)
(72) Erfinder: SANDMEIER, Dieter, 91080 Uttenreuth (DE); KENSBOCK, Eva, 81375 München (DE)
(86) Internationale Anmeldenummer: PCT/EP2006/002701
(87) Internationale Veröffentlichungsnummer: WO 2006/133754

(56) Entgegenhaltungen:
- WO-A-2004/052961
- US-A1- 2004 219 128
- US-B1- 6 403 113
- US-B1- 6 506 737

## Beschreibung

### Technisches Gebiet

Die Erfindung bezieht sich auf ein Verfahren zur Herstellung einer antimikrobiell wirkenden Beschichtung auf einer technischen Oberfläche. Das Verfahren findet Anwendung in der Lebensmittel- und Verpackungsmittelindustrie, insbesondere zur Verpackung verderblicher Lebensmittel. Darüber hinaus ist der Einsatz des Verfahrens in allen Bereichen möglich, in denen es gilt Oberflächenbereiche vor einer Kontamination mit Bakterien, Pilzen, Viren, Ricksettien oder Keimen zu schützen, wie bspw. in der Medizin, der Pharmazie, der Biologie, bei Gebrauchsgütern, im Haushalt oder in der Textilindustrie.

### Stand der Technik

Beschichtungen der vorstehend genannten Gattung werden in an sich bekannter Weise bei Lebensmittelverpackungen zum Schutz der verpackten Lebensmittel vor Befall durch Mirkoorganismen, wie Bakterien, Pilzen oder sonstige Keime eingesetzt. Insbesondere verderbliche Lebensmittel können durch die Verwendung antimikrobiell beschichteter Verpackungsmaterialien längere Zeit in einem qualitativ hochwertigen Zustand gelagert werden. Zusätzlich wird das Risiko des Entstehens von Krankheitserregern an oder in den verpackten Lebensmitteln reduziert. Antimikrobiell wirkende Beschichtungen weisen typischerweise eine matrixartig ausgebildete Trägerschicht mit darin eingelagerten Wirk- oder Hemmstoffen auf, die, sofern sie in Kontakt mit Mikroorganismen treten, diese abtöten oder deren Wachstum hemmen oder unterbinden. Derzeit sind grundsätzlich zwei Funktionsprinzipien antimikrobiell wirkender Schichten bekannt:

Das erste Funktionsprinzip ist dadurch gekennzeichnet, dass die antimikrobiellen Wirk- und Hemmstoffe volatile, d.h. flüchtige Eigenschaften besitzen und zunächst in einer matrixartig ausgebildeten Trägerschicht eingelagert sind, aber mit der Zeit von dem Matrixmaterial freigesetzt werden. Die antimikrobielle Wirkung entsteht demzufolge nicht nur im Nahbereich über der beschichteten Oberfläche, sondern die Wirk- und Hemmstoffe können durch Freisetzung aus der Trägerschicht im gesamten Verpackungsvolumen ihre antimikrobielle Wirkung entfalten. Geeignete antimikrobielle Wirkstoffe für dieses Funktionsprinzip sind für Lebensmittelanwendungen im europäischen Raum nicht einsetzbar (bspw. Chlordioxid da toxisch oder Ethanol da nachteilige sensorischen Eigenschaften aufweisend).

Das zweite Funktionsprinzip ist bedeutend weiter verbreitet und zeichnet sich dadurch aus, dass die antimikrobiell wirkenden Wirk- und Hemmstoffe in der Trägerschicht derart räumlich fixiert sind, dass sie aus der Trägerschicht nicht selbständig entweichen können. Die antimikrobielle Wirkung der in das matrixartig ausgebildete Schichtmaterial eingelagerten Wirk- und Hemmstoffe entfaltet sich insbesondere durch Kontakt der Lebensmittel mit der antimikrobiell beschichteten Oberfläche. Dabei können die Wirk- und Hemmstoffe durch die Oberfläche des Matrixmaterials in die Oberfläche des Lebensmittels diffundieren, so dass die antimikrobielle Wirkung der Beschichtung wesentlich durch die Diffusionskinetik bestimmt wird.

Im Hinblick auf die Verpackung von Lebensmitteln mit antimikrobiell beschichteten Lebensmittelverpackungen sind in den vergangenen Jahren eine Vielzahl von Wirk-und Hemmstoffe sowie Release Mechanismen erforscht worden. Einen Überblick hierzu vermittelt die Veröffentlichung: ,Review of antimicrobial food packaging' von Paola Appendini und Joseph H. Hotchkiss in Innovative Food Science & Emerging Technologies 3 (2002) 113 - 126.

Die bisher bekannten Verfahren zur Herstellung einer antimikrobiell wirkenden Beschichtung haben jedoch den Nachteil, dass sie entweder technisch aufwendig herzustellen und daher teuer sind, oder die verwendeten Ausgangsstoffe nicht in ausreichender Menge zur Verfügung stehen oder Ausgangsstoffe und/ oder die resultierenden Produktstoffe nicht den gesetzlichen Bestimmungen genügen.

In der WO 2004 /052961 A wird ein Verfahren zur Herstellung von Copolymeren auf Hexamethylendiamin- und Guanidin-Basis beschrieben, die über sterilisierende Eigenschaften verfügen und in Form eines Beschichtungsmaterials auf zu schützende Oberflächen aufgebracht werden können.

In der US 2004/219128 A1 sind desinfizierende, antimikrobielle Dichtungszusammensetzungen sowie deren Anwendungen beschrieben. Insbesondere dienen die in dieser Druckschrift beschriebenen Zusammensetzungen zur dauerhaften Abscheidung an Oberflächen, die es gilt keimfrei zu halten.

### Darstellung der Erfindung

Der Erfindung liegt die Aufgabe zugrunde ein möglichst kostengünstiges Verfahren zur Herstellung einer antimikrobiell wirkenden Beschichtung auf einer technischen Oberfläche anzugeben, das technisch einfach ist und den bestehenden gesetzlichen Auflagen genügt. Die Erfindung soll insbesondere anwendbar sein zur Herstellung antimikrobiell wirkender Beschichtungen auf Lebensmittelverpackungen sowie auf technischen Oberflächen, die von Relevanz sind in der Medizin, der Pharmazie, der Biologie, bei Gebrauchsgütern, im Haushalt oder in der Textilindustrie und die vor einer Besiedelung von Mikroorganismen wie bspw. Bakterien, Pilzen oder Keimen geschützt werden sollen. Das Verfahren soll zudem mit konventionellen Anlagen durchführbar sein, so dass auf bereits im Einsatz befindliche Beschichtungssysteme zurückgegriffen werden kann.

Die Lösung der der Erfindung zugrunde liegenden Aufgabe ist in Anspruch 1 angegeben. Den Unteransprüchen sowie der weiteren Beschreibung sind vorteilhafte Merkmale des Erfindungsgedankens zu entnehmen.

Das erfindungsgemäße Verfahren zeichnet sich im Wesentlichen durch die drei Verfahrensschritte aus:
a) Herstellen einer Lösung aus Polyvinylacetat, einem Konservierungsmittel und einem Lösungsmittel, bei der als Konservierungsmittel antimikrobiell wirkende Stoffe oder Stoffgemische, insbesondere Benzoesäure, Sorbinsäure, Natamycin, Bacteriocine, Pflanzenextrakte oder deren Gemische, und als Lösungsmittel ein Ethanol-Wasser Gemisch oder Ethylacetat oder Aceton verwendet werden, und der Lösung als Aktivitätsregulator Milchsäure oder Zitronensäure zugegeben wird,
b) Aufbringen der Lösung auf die technische Oberfläche, und
c) Trocknen der auf die technische Oberfläche aufgebrachten Lösung unter Ausbildung der Schicht.

In dem ersten Verfahrensschritt wird eine Lösung hergestellt, die die Komponenten Polyvinylacetat, ein Konservierungsmittel und ein Lösungsmittel aufweist. Als Lösungsmittel wird 100% Ethylacetat oder vorzugsweise ein Ethanol-Wassergemisch mit einem Mischungsverhältnis Ethanol/ Wasser zwischen 80: 20 bis 100: 0 Gewichtsprozent verwendet. Das Ethanol-Wassergemisch dient als technisches, physiologisches und umweltverträgliches Lösungsmittel, das verdampft und größtenteils wieder zurück gewonnen werden kann. Darüber hinaus eignet sich Aceton ebenfalls als Lösemittel.

Dem Lösungsmittel wird zunächst Polyvinylacetat zugegeben und im Lösungsmittel vollständig gelöst. Polyvinylacetat hat sich als idealer Stoff für die matrixartige Trägerschicht der herzustellenden antimikrobiellen Beschichtung erwiesen, da es einfach in einem Lösungsmittel löslich ist, als Trägerschicht sehr gute ,Release'-Eigenschaften für die darin eingelagerten antimikrobiellen Wirk- oder Hemmstoffe aufweist und nach dem Aufbringen auf eine technische Oberfläche eine transparente und flexible, filmartige Trägeschicht ausbildet. Die Lösung enthält vorzugsweise bis zu 60 Gewichtsprozent Polyvinylacetat, bezogen auf die Menge an Lösungsmittel.

Anschließend wird dem Lösungsmittel das Konservierungsmittel zugegeben. Das Konservierungsmittel entspricht dem eigentlich antimikrobiell wirkenden Wirk- oder Hemmstoff. Grundsätzlich ist eine Vielzahl derartiger Wirk- oder Hemmstoffe bekannt, die in dem vorliegenden Verfahren Verwendung finden können (vgl. Paola Appendini und Joseph H. Hotchkiss, Innovative Food Science & Emerging Technologies 3 (2002) Seite 115, Tabelle 2). Als besonders geeignete Wirk- oder Hemmstoffe haben sich Benzoesäure, Sorbinsäure, Natamycin, Bacteriocine (bspw. Nisin oder Pediocin), Pflanzenextrakte oder deren Gemische erwiesen. Insbesondere Benzoesäure und Sorbinsäure sind rechtlich sowohl für Lebensmittel wie auch für Kunststoffe zur Lebens-mittelverpackung zugelassen und weisen ein breites antimikrobielles Wirkspektrum gegenüber unterschiedlichen Gruppen von Mikroorganismen, wie beispielsweise Bakterien, Hefen oder Schimmelpilzen, auf. In Polyvinylacetatfilmen bleiben diese beiden Stoffe zudem bis in hohe Konzentrationsbereiche gelöst, ohne dass sie die Transparenz der Polyvinylacetatfilme durch eigene Kristallbildung beeinflussen. Es können jedoch, wie angegeben, auch andere bekannte, lebensmittelrechtlich zugelassene Konservierungsstoffe bzw. deren Gemische verwendet werden. Vorzugsweise enthält die Lösung mit den Komponenten Lösungsmittel, Polyvinylacetat und Konservierungsmittel bis zu 40 Gewichtsprozent Konservierungsstoff, bezogen auf die in der Lösung enthaltene Menge an Polyvinylacetat.

Desweiteren wird der Lösung ein so genannter Aktivitätsregulator zugegeben, der die Funktion hat, den pH-Wert der technischen Oberfläche und deren nächster Umgebung derart einzustellen, dass das oder die Konservierungsmittel in der Lösung in undissozüerter Form vorliegen. Die Konservierungsmittel, die wie vorstehend erwähnt, den Wirk- oder Hemmstoffen entsprechen, können im undissoziierten Zustand ihre antimikrobielle Wirkung bestmöglich entfalten. Die Verwendung von Milchsäure oder Zitronensäure als Aktivitätsregulator hat sich überraschender Weise in Kombination mit Benzoe- und/ oder Sorbinsäure als besonders geeignet erwiesen. Vorzugsweise enthält die Lösung bis zu 5 Gewichtsprozent des Aktivitätsregulators, bezogen auf die in der Lösung enthaltene Menge an Konservierungsmittel.

Das Herstellen der Lösung erfolgt vorzugsweise unter ständigem Rühren und ggf. unter Einhaltung eines zur Herstellung der Lösung idealen Temperaturniveaus im Bereich zwischen 20 - 50°C.

Im zweiten Verfahrensschritt wird die vermischte und ggf. temperierte Lösung auf die technische Oberfläche aufgetragen. Hierfür kommen grundsätzlich alle für den gezielten Auftrag eines flüssigen Mediums auf eine technische Oberfläche zu Verfügung stehenden Methoden und Techniken in Betracht, wie beispielsweise Tauch-, Druck-, Lackier- oder Sprühverfahren sowie das Aufbringen der Lösung mittels Pinseln, Rakeln oder Riffelwalzen. Die zum Aufbringen der Lösung auszuwählende Technik richtet sich nach der Art der Beschaffenheit der zu beschichtenden Oberfläche, bzw. der Form des die Oberfläche aufweisenden Körpers. Zur Beschichtung bspw. von Polymerfolien oder Papier können übliche Lackierverfahren zum Einsatz kommen, wohingegen bei becherförmigen Behältnissen Sprühtechniken besser geeignet sind.

Als besonders vorteilhaft ist erkannt worden, dass sich technische Oberflächen mit einer bevorzugten elektrischen Oberflächenpolarität für einen innigen Verbund zwischen Oberfläche und Lösung eignen. Die elektrische Polarität von technischen Oberflächen kann durch elektrische Felder erzeugt oder verändert werden. Vorzugsweise erfolgt das elektrische Polarisieren der technische Oberfläche vor dem Aufbringen der Lösung mittels Coronaentladung.

Im dritten Verfahrensschritt erfolgt eine Trocknung der auf die technische Oberfläche aufgebrachten Lösung bei Temperaturen unter 60°C. Durch die Trocknung verflüchtigt sich das Lösungsmittel unter Ausbildung der aus Polyvinylacetat bestehenden matrixartigen Trägerschicht mit den darin eingelagerten Konservierungsmitteln, d.h. den antimikobiell wirkenden Wirk- oder Hemmstoffen.

Mit dem erfindungsgemäßen Verfahren ist somit eine antimikrobiell wirkende Beschichtung auf einer technischen Oberfläche herstellbar, die als Trägerschicht Polyvinylacetat mit einem oder mehreren darin eingelagerten antimikrobiell wirkenden Wirk- oder Hemmstoffen aufweist, die mit der Zeit vom Matrixmaterial freigesetzt werden. Als technische Oberflächen kommen hierfür Oberflächen von Lebensmittelverpackungen, insbesondere Folienoberflächen und/oder von aus Kunststoff, Papier, Metall oder Naturstoffen gefertigten Behältnissen in Betracht. Darüber hinaus ist die erfindungsgemäße Beschichtung auch für technische Oberflächen in den Bereichen der Medizin, der Pharmazie, der Biologie, bei Gebrauchsgütern, im Haushalt oder in der Textilindustrie anwendbar, die vor einer Besiedelung von Mikroorganismen wie bspw. Bakterien, Pilzen oder anderen Keimen geschützt werden sollen.

Mit dem erfindungsgemäßen Verfahren ist es zudem möglich, mit einfachen, konventionellen technischen Mitteln, unter Verwendung von kostengünstigen und in beliebiger Menge verfügbaren, lebensmittelrechtlich zugelassenen Ausgangsstoffen, antimikrobiell wirkende Beschichtungen auf technische Oberflächen aufzutragen.

Die Erfindung wird nachstehend ohne Beschränkung des allgemeinen Erfindungsgedankens anhand eines nachfolgenden Ausführungsbeispieles beschrieben.

Zunächst wird aus 10 g Polyvinylacetat, das in Pulverform oder als Granulat erhältlich ist, und 100 g eines Lösungsmittels, bestehend aus einer Mischung aus Ethanol und Wasser mit einem Mischungsverhältnis von 95 : 5 Gew. %, unter Rühren und Erwärmen auf 30 - 40 °C eine klare Lösung hergestellt, so dass das Polyvinylacetat in vollständig gelöster Form vorliegt. Anschließend werden in die Lösung 10 Gew. % Sorbinsäure (Konservierungsmittel), bezogen auf das in der Lösung enthaltene Polyvinylacetat, d.h. 1 g Sorbinsäure, und 0,1 Gew. % Milchsäure, bezogen auf die in der Lösung enthaltene Menge Konservierungsmittel, d.h. 0,001g Milchsäure, zugegeben. Nach kurzer Zeit bildet sich eine klare Lösung aus. Die Lösung wird nun auf eine aus Polyolefinen bestehende Trägerfolie aufgebracht und mittels eines Rakels auf deren Oberfläche verteilt. Abhängig vom jeweils verwendeten Rakel wurden dabei Schichtdicken der aufgebrachten Lösung zwischen 40 - 100 µm erzeugt. Zur besseren Haftung der aufgebrachten Lösung auf der Trägerfolie ist es vorteilhaft, vor dem Auftragen der Lösung, mittels Coronaverfahren eine Oberflächenspannung von ca. 40 dyn/cm² auf der Trägerfolienoberfläche zu erzeugen. Dies führt insbesondere bei der hier verwendeten Trägerfolle aus Polyolefinen zu erheblich verbesserten Haftungseigenschaften zwischen der Folie und der aufgetragenen Lösung.

Die auf die Trägerfolie übertragene Lösung wird nun bei einer Temperatur von 50°C ca. 5 Minuten getrocknet. Je nach angewendetem Trocknungsprozess sind weitere Parameter wie bspw. die zugeführte Luftmenge oder die Luftfeuchte zu berücksichtigen. Als Temperaturlimit sollte während des Trocknungsprozesses eine Temperatur von 60°C nicht überschritten werden. Während der Trocknung bildet sich auf der Polymerfolie eine transparente, flexible Schichtung aus. Nach dem Abkühlen kann deren antimikrobielle Wirkung beispielsweise im einem modifizierten Agar-Diffusionstest überprüft werden, der folgende vier Schritte umfasst:
1. Bereitstellen einer Petrischale mit einer sterilen Agarschicht,
2. Auflegen der Testfolie mit beschichteten und unbeschichteten Bereichen auf die Agarschicht,
3. Aufbringen von Agar, der einen Testkeim enthält, auf die Testfolie,
4. Bebrüten und Auswerten.

Die antimikrobiell wirkende, d.h. mit der Lösung beschichtete Polyolefinfolie zeigt in dem beschriebenen Agar-Diffuionstest unter Verwendung des Testorganismus Saccharomyces cerevisiae, dass in dem beschichteten Oberflächenbereich der Testfolie keine Ansiedlungen von Mikroorganismen auftreten. Auch gegenüber Mikroorganismen aus der Gattung der Bakterien, grampositiv wie gramnegativ, aus der Gattung der Pilze und aus anderen Bereichen der Mikrobiologie zeigen beschichtete Folien, die nach dem oben beschriebenen Verfahren hergestellt wurden, ihre hemmende Wirkung. Durch Messungen ergab sich weiter, dass die untersuchten Beschichtungen dabei in 2 Tagen ca. 20 - 40 mg/ m² der verwendeten Konservierungsstoffe freisetzen.

## Patentansprüche

1. Verfahren zur Herstellung einer antimikrobielle Wirkstoffe freisetzenden Schicht auf einer technischen Oberfläche,
**gekennzeichnet durch** folgende Verfahrensschritte:
- Herstellen einer Lösung aus Polyvinylacetat, einem Konservierungsmittel und einem Lösungsmittel, bei der als Konservierungsmittel antimikrobiell wirkende Stoffe oder Stoffgemische, insbesondere Benzoesäure, Sorbinsäure, Natamycin, Bacteriocine, Pflanzenextrakte oder deren Gemische, und als Lösungsmittel ein Ethanol-Wasser Gemisch oder Ethylacetat oder Aceton verwendet werden, und der Lösung als Aktivitätsregulator Milchsäure oder Zitronensäure zugegeben wird
- Aufbringen der Lösung auf die technische Oberfläche, und
- Trocknen der auf die technische Oberfläche aufgebrachten Lösung unter Ausbildung der Schicht.

2. Verfahren nach Anspruch 1,
**dadurch gekennzeichnet, dass** die Lösung bis 60 Gew. % Polyvinylacetat, bezogen auf das Lösungsmittel, enthält.

3. Verfahren nach Anspruch 1 oder 2,
**dadurch gekennzeichnet, dass** die Lösung bis 40 Gew. % Konservierungsmittel, bezogen auf das in der Lösung enthaltene Polyvinylacetat enthält.

4. Verfahren nach einem der Ansprüche 1 bis 3,
**dadurch gekennzeichnet, dass** die Lösung bis 5 Gew. % Aktivitätsregulator, bezogen auf das in der Lösung enthaltene Konservierungsmittel, enthält.

5. Verfahren nach einem der Ansprüche 1 bis 3,
**dadurch gekennzeichnet, dass** das Lösungsmittel aus einem Ethanol-Wassergemisch mit einem Mischungsverhältnis von 80 : 20 bis 100 : 0 Gew. % besteht.

6. Verfahren nach einem der Ansprüche 1 bis 5,
**dadurch gekennzeichnet, dass** das Herstellen der Lösung bei Temperaturen von 20° - 50°C durch Mischen erfolgt.

7. Verfahren nach einem der Ansprüche 1 bis 6,
**dadurch gekennzeichnet, dass** die Lösung durch Tauch-, Druck-, Lackier- oder Sprühverfahren, oder mittels Pinsein, Rakeln oder Riffelwalzen auf die technische Oberfläche aufgebracht wird.

8. Verfahren nach einem der Ansprüche 1 bis 7,
**dadurch gekennzeichnet, dass** als technische Oberfläche eine Oberfläche eines Mittels zur Lebensmittelaufbewahrung, insbesondere einer Lebensmittelverpackung, eines Bedarfsgegenstandes oder eines im medizinischen Bereich eingesetzten Mittels verwendet wird.

9. Verfahren nach Anspruch 8,
**dadurch gekennzeichnet, dass** als Mittel zur Lebensmittelaufbewahrung eine Folie und/ oder aus Kunststoff, Papier, Metall oder Naturstoffen gefertigte Behältnisse verwendet werden.

10. Verfahren nach einem der Ansprüche 1 bis 9,
**dadurch gekennzeichnet, dass** vor dem Aufbringen der Lösung auf die technische Oberfläche, die technische Oberfläche elektrisch polarisiert wird.

11. Verfahren nach Anspruch 10,
**dadurch gekennzeichnet, dass** die elektrische Polarisierung mittels CoronaVerfahren erzeugt wird.

12. Verfahren nach einem der Ansprüche 1 bis 11,
**dadurch gekennzeichnet, dass** das Trocknen der auf die Oberfläche aufgebrachten Lösung bei Temperaturen kleiner 60°C erfolgt.

## Claims

1. Method for producing a layer, which releases antimicrobial substances, on a technical surface,
**characterised by** the following method steps:
- production of a solution made of polyvinyl acetate, a preservative and a solvent, in which there are used, as preservative, substances or substance mixtures which act antimicrobially, in particular benzoic acid, sorbic acid, natamycin, bacteriocins, plant extracts or mixtures thereof and, as solvent, an ethanol-water mixture or ethyl acetate or acetone, and lactic acid or citric acid is added to the solution as activity regulator,
- application of the solution on the technical surface, and
- drying of the solution applied on the technical surface with formation of the layer.

2. Method according to claim 1,
**characterised in that** the solution comprises up to 60% by weight of polyvinyl acetate, relative to the solvent.

3. Method according to claim 1 or 2,
**characterised in that** the solution comprises up to 40% by weight of preservative, relative to the polyvinyl acetate contained in the solution.

4. Method according to one of the claims 1 to 3,
**characterised in that** the solution comprises up to 5% by weight of activity regulator, relative to the preservative contained in the solution.

5. Method according to one of the claims 1 to 3,
**characterised in that** the solvent consists of an ethanol-water mixture with a mixture ratio of 80 : 20 to 100 : 0% by weight.

6. Method according to one of the claims 1 to 5,
**characterised in that** the production of the solution at temperatures of 20° - 50°C is effected by mixing.

7. Method according to one of the claims 1 to 6,
**characterised in that** the solution is applied on the technical surface by dipping, printing, painting or spraying methods or by means of brushes, doctor blades or corrugated rollers.

8. Method according to one of the claims 1 to 7,
**characterised in that** a surface of a means for foodstuff preservation, in particular of foodstuff packaging, of a commodity or of a means used in the medical sphere is used as technical surface.

9. Method according to claim 8,
**characterised in that** a film and/or containers manufactured from plastic material, paper, metal or natural materials are used as means for foodstuff preservation.

10. Method according to one of the claims 1 to 9,
**characterised in that**, prior to the application of the solution on the technical surface, the technical surface is electrically polarised.

11. Method according to claim 10,
**characterised in that** the electrical polarisation is produced by means of a corona method.

12. Method according to one of the claims 1 to 11,
**characterised in that** the drying of the solution applied on the surface is effected at temperatures of less than 60°C.

## Revendications

1. Procédé d'obtention d'une couche libérant un agent actif antimicrobien sur une surface technique,
procédé **caractérisé par** les étapes suivantes :
- préparation d'une solution de polyvinyl acétate, d'un agent de conservation et d'un solvant, selon laquelle, en tant qu'agent de conservation, on met en oeuvre des matériaux ou mélanges de matériaux à effet antimicrobien, en particulier l'acide benzoïque, l'acide sorbique, la natamycine, la bactériocine, des extraits de plantes ou leurs mélanges, et en tant que solvant, on met en oeuvre un mélange éthanol-eau, ou de l'acétate d'éthyle ou de l'acétone, et on ajoute à la solution en tant que régulateur d'activité de l'acide lactique ou de l'acide citrique,
- application de la solution sur la surface technique, et
- séchage de la solution appliquée sur la surface technique avec formation de la couche.

2. Procédé conforme à la revendication 1,
**caractérisé en ce que**
la solution renferme jusqu'à 60 % en poids de polyvinyl acétate par rapport au solvant.

3. Procédé conforme à la revendication 1 ou à la revendication 2, **caractérisé en ce que**
la solution renferme jusqu'à 40 % en poids d'agent de conservation par rapport au polyvinyl acétate contenu dans la solution.

4. Procédé conforme à l'une des revendications 1 à 3,
**caractérisé en ce que**
la solution renferme jusqu'à 5 % en poids de régulateur d'activité par rapport à l'agent de conservation contenu dans la solution.

5. Procédé conforme à l'une des revendications 1 à 3,
**caractérisé en ce que**
le solvant consiste en un mélange éthanol-eau dans un rapport de mélange de 80:20 à 100:0 % en poids.

6. Procédé conforme à l'une des revendications 1 à 5,
**caractérisé en ce que**
la préparation de la solution s'effectue par mélange à des températures de 20-50°C.

7. Procédé conforme à l'une des revendications 1 à 6,
**caractérisé en ce que**
la solution est appliquée sur la surface technique par un procédé d'immersion d'impression d'enduction ou de pulvérisation ou à l'aide de pinceaux, de raclettes ou de cylindres cannelés.

8. Procédé conforme à l'une des revendications 1 à 7,
**caractérisé en ce qu'**
en tant que surface technique, on utilise une surface d'un moyen pour la conservation d'un aliment, en particulier un emballage alimentaire, un objet utilitaire ou un moyen mis en oeuvre dans le domaine médical.

9. Procédé conforme à la revendication 8,
**caractérisé en ce qu'**
en tant que moyens pour la conservation d'un aliment, on utilise une feuille et/ou un objet réalisé en matériau synthétique, en papier, en métal, ou en matériau naturel.

10. Procédé conforme à l'une des revendications 1 à 9,
**caractérisé en ce qu'**
avant l'application de la solution sur la surface technique, on polarise cette surface électriquement.

11. Procédé conforme à la revendication 10,
**caractérisé en ce qu'**
on effectue la polarisation électrique par procédé Corona.

12. Procédé conforme à l'une des revendications 1 à 11,
**caractérisé en ce qu'**
on effectue le séchage de la solution appliquée sur la surface à des températures inférieures à 60°C.
